# EUROPÄISCHE PATENTSCHRIFT

(11) **EP 0 410 208 B1**
(45) Veröffentlichungstag und Bekanntmachung des Hinweises auf die Patenterteilung: **22.06.1994**
(21) Anmeldenummer: 90113218.3
(22) Anmeldetag: 11.07.1990
(51) Int. Cl.: C07D 311/68, C07D 405/04, C07D 405/12, A61K 31/44

(54) **Chromanderivate**
Chroman derivatives
Dérivés de chromane

(30) Priorität: 24.07.1989 DE 3924417
(43) Veröffentlichungstag der Anmeldung: 30.01.1991
(73) Patentinhaber: MERCK PATENT GmbH, D-64271 Darmstadt (DE)
(72) Erfinder: Gericke, Rolf, Dr., D-6104 Seeheim (DE); Baumgarth, Manfred, Dr., D-6100 Darmstadt (DE); Lues, Ingeborg, Dr., D-6100 Darmstadt (DE); Bergmann, Rolf, Dr., D-6101 Reichelsheim (DE); De Peyer, Jacques, Dr., CH-3007 Bern (CH)

(56) Entgegenhaltungen:
- EP-A- 0 172 352
- EP-A- 0 298 452
- EP-A- 0 312 432
- EP-A- 0 363 883
- GB-A- 2 204 868

## Beschreibung

Die Erfindung betrifft neue Chromanderivate der Formel I 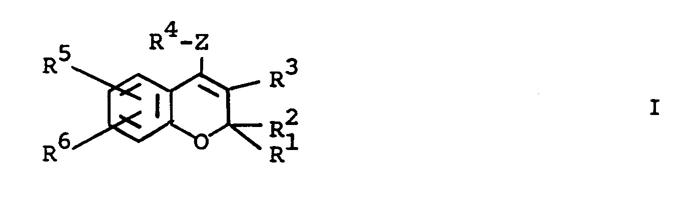
worin
- R¹: A,
- R²: H oder A,
- R¹ und R²: zusammen auch Alkylen mit 3-6 C-Atomen,
- R³: CHO oder CH₂OH,
- R⁴: einen unsubstituierten oder ein- oder zweifach durch A, F, Cl, Br, J, OH, OA, OAc, SH, NO₂, NH₂, AcNH, COOH und/oder COOA substituierten Pyridyl-, Pyridazinyl-, Pyrimidinyl-, Pyrazinyl-, Oxo-dihydro-pyridyl-, Oxo-dihydro-pyridazinyl-, Oxo-dihydro-pyrimidinyl-, Oxo-dihydro-pyrazinyl-, 2-Pyrrolidinon-1-yl- oder 3-Oxo-cyclopenten-1-yl-rest,
- R⁵ und R⁶: jeweils H, A, HO, AO, CHO, ACO, ACS, HOOC, AOOC, AO-CS, ACOO, A-CS-O, Hydroxy-alkyl, Mercapto-alkyl, NO₂, NH₂, NHA, NA₂, CN, F, Cl, Br, J, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitro-alkyl, Cyan-alkyl, A-C(=NOH) oder A-C(=NNH₂),
- Z: O, S, NH oder eine Bindung,
- A: Alkyl mit 1-6 C-Atomen,
- -alkyl: Alkylen mit 1-6 C-Atomen und
- Ac: Alkanoyl mit 1-8 C-Atomen oder Aroyl mit 7-11 C-Atomen
bedeuten,
sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z.B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z.B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls R¹ und R² zusammen Alkylen bedeuten, so ist die Alkylengruppe vorzugsweise unverzweigt, im einzelnen bevorzugt -(CH₂)ₙ-, wobei n 3, 4, 5 oder 6 bedeutet.

Die Gruppe "-alkyl" steht vorzugsweise für -CH₂- oder -CH₂CH₂-.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

R¹ und R² sind vorzugsweise jeweils Alkyl, insbesondere jeweils Methyl oder Ethyl, bevorzugt jeweils Methyl; weiterhin sind R¹ und R² zusammen bevorzugt -(CH₂)₄-oder -(CH₂)₅-.

Z bedeutet vorzugsweise O oder eine Bindung.

Die Gruppe R⁴-Z ist vorzugsweise 1,2-Dihydro-2-oxo-1-pyridyl, ferner 2-Hydroxy-4-pyridyl-oxy (= 1,2-Dihydro-2-oxo-4-pyridyl-oxy), 6-Hydroxy-3-pyridazinyl-oxy (1,6-Dihydro-6-oxo-3-pyridazinyl-oxy), 2-, 3- oder 4-Pyridyl-oxy, 1,6-Dihydro-3-hydroxy-6-pyridazinon-1-yl, 1,2-Dihydro-4-hydroxy-2-oxo-1-pyridyl, 1,6-Dihydro-1-methyl-6-oxo-3-pyridazinyl-oxy, 1,6-Dihydro-1-ethyl-6-oxo-3-pyridazinyl-oxy, 2-Pyrrolidinon-1-yl oder 3-Oxo-cyclopenten-1-yl-oxy.

In R⁵ und R⁶ bedeuten vorzugsweise:
- A:: Methyl, ferner Ethyl;
- AO:: Methoxy, ferner Ethoxy;
- ACO:: Acetyl, ferner Propionyl;
- ACS:: Thioacetyl, ferner Thiopropionyl;
- AOOC:: Methoxycarbonyl, ferner Ethoxycarbonyl;
- AO-CS:: Methoxy-thiocarbonyl, ferner Ethoxythiocarbonyl;
- ACOO:: Acetoxy, ferner Propionoxy;
- ACSO:: Thio(no)acetoxy, ferner Thio(no)propionoxy; Hydroxy-alkyl: Hydroxymethyl oder 1- oder 2-Hydroxyethyl; Mercapto-alkyl:Mercaptomethyl oder 1- oder 2-Mercaptoethyl;
- NHA:: Methylamino, ferner Ethylamino;
- NA₂:: Dimethylamino, ferner Diethylamino;
- ASO:: Methylsulfinyl, ferner Ethylsulfinyl;
- ASO₂:: Methylsulfonyl, ferner Ethylsulfonyl;
- AO-SO:: Methoxy-sulfinyl, ferner Ethoxy-sulfinyl;
- AO-SO₂:: Methoxy-sulfonyl, ferner Ethoxy-sulfonyl;
- Ac-NH:: Acetamido, ferner Formamido, Propionamido oder Benzamido;
- AO-CO-NH:: Methoxycarbonylamino, ferner Ethoxycarbonylamino;
- HANSO:: Methylaminosulfinyl, ferner Ethylaminosulfinyl
- A₂NSO:: Dimethylaminosulfinyl, ferner Diethylaminosulfinyl;
- HANSO₂:: Methylaminosulfonyl, ferner Ethylaminosulfonyl;
- A₂NSO₂:: Dimethylaminosulfonyl, ferner Diethylaminosulfonyl;
- HANCO:: N-Methylcarbamoyl, ferner N-Ethylcarbamoyl;
- A₂NOC:: N,N-Dimethylcarbamoyl, ferner N,N-Diethylcarbamoyl;
- HANCS:: N-Methyl-thiocarbamoyl, ferner N-Ethylthiocarbamoyl;
- A₂NCS:: N,N-Dimethyl-thiocarbamoyl, ferner N,N-Diethyl-thiocarbamoyl;
- ASONH:: Methylsulfinylamino, ferner Ethylsulfinylamino;
- ASO₂NH:: Methylsulfonylamino, ferner Ethylsulfonylamino;
- AOSONH:: Methoxysulfinylamino, ferner Ethoxysulfinylamino;
- AOSO₂NH:: Methoxysulfonylamino, ferner Ethoxysulfonylamino;
- ACO-alkyl:: 2-Oxopropyl, 2-Oxobutyl, 3-Oxobutyl, 3-Oxopentyl;
- Nitro-alkyl:: Nitromethyl, 1- oder 2-Nitroethyl; Cyan-alkyl: Cyanmethyl, 1- oder 2-Cyanethyl;
- A-C(= NOH):: 1-Oximinoethyl, ferner 1-Oximinopropyl;
- A-C(= NNH₂):: 1-Hydrazonoethyl, ferner 1-Hydrazonopropyl.

Die Reste R⁵ und R⁶ stehen vorzugsweise in 6- und 7-Stellung des Chromansystems. Sie können jedoch auch in 5- und 6-, 5- und 7-, 5- und 8-, 6- und 8- sowie 7- und 8-Stellung stehen.

Von den Resten R⁵ und R⁶ ist vorzugsweise der eine H, während der andere von H verschieden ist. Dieser andere Rest steht vorzugsweise in 6-Stellung, aber auch in 5-, 7- oder 8-Stellung, und ist vorzugsweise CN oder NO₂, ferner bevorzugt CHO, ACO (insbesondere Acetyl), AOOC (insbesondere Methoxycarbonyl oder Ethoxycarbonyl), ACOO (insbesondere Acetoxy), weiterhin bevorzugt F, Cl, Br, J, CF₃, H₂NCO, H₂NCS oder NH₂.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ii ausgedrückt werden, die der Formel I entsprechen und worin die nicht näher bezeichneten Reste die bei der Formel I angegebene Bedeutung haben, worin jedoch
in Ia
- R¹ und R²: jeweils A bedeuten;
in Ib
- R¹ und R²: jeweils CH₃ bedeuten;
in Ic
- R¹ und R²: zusammen Alkylen mit 3-6 C-Atomen bedeuten;
in Id
- R⁴-Z: 1,2-Dihydro-2-oxo-1-pyridyl, 2-Hydroxy-4-pyridyl-oxy, 6-Hydroxy-3-pyridazinyl-oxy, 1,6-Dihydro-1-methyl-6-oxo-3-pyridazinyl-oxy, 2-Pyrrolidinon-1-yl oder 3-Oxo-cyclopenten-1-yl-oxy bedeutet;
in Ie
- R⁴-Z: 1,2-Dihydro-2-oxo-1-pyridyl bedeutet;
in If
- R¹ und R²: jeweils CH₃ oder zusammen -(CH₂)₄-oder -(CH₂)₅- und
- R⁴-Z: 1,2-Dihydro-2-oxo-1-pyridyl, 2-Hydroxy-4-pyridyl-oxy, 6-Hydroxy-3-pyridazinyl-oxy, 1,6-Dihydro-1-methyl-6-oxo-3-pyridazinyl-oxy, 2-Pyrrolidinon-1-yl oder 3-Oxo-cyclopenten-1-yl-oxy bedeuten;
in Ig
- R¹ und R²: jeweils CH₃ und
- R⁴-Z: 1,2-Dihydro-2-oxo-1-pyridyl oder 6-Hydroxy-3-pyridazinyl-oxy bedeuten,
in Ih
- R¹ und R²: jeweils CH₃ und
- R⁴-Z: 2-Pyrrolidinon-1-yl bedeuten;
in Ii
- R¹ und R²: jeweils CH₃ und
- R⁴-Z: 1,2-Dihydro-2-oxo-1-pyridyl bedeuten.

Weiterhin sind bevorzugt Verbindungen der Formeln I′ sowie Ia′ bis Ii′, die den Formeln I sowie Ia bis Ii entsprechen, worin jedoch jeweils zusätzlich R³ CHO bedeutet.

Weiterhin sind bevorzugt Verbindungen der Formeln I˝ sowie Ia˝ bis Ii˝, die den Formeln I sowie Ia bis Ii entsprechen, worin jedoch jeweils zusätzlich R³ CH₂OH bedeutet.

Ferner sind bevorzugt Verbindungen der Formeln I, I′, I˝, Ia bis Ii, Ia′ bis Ii′ sowie Ia˝ bis Ii˝, worin jeweils zusätzlich.
(a)
   - R⁵: von H verschieden ist und
   - R⁶: H bedeutet;
(b)
   - R⁵: von H verschieden ist und in 6-Stellung steht und
   - R⁶: H bedeutet;
(c)
   - R⁵: NO₂, CN, CHO, ACO, HOOC, AOOC, ACOO, F, Cl, Br, J, CF₃, H₂NCO, H₂NCS oder NH₂ und
   - R⁶: H bedeutet;
(d)
   - R⁵: NO₂, CN, CHO, ACO, HOOC, AOOC, ACOO, F, Cl, Br, J, CF₃, H₂NCO, H₂NCS oder NH₂ bedeutet und in 6-Stellung steht und
   - R⁶: H bedeutet;
(e)
   - R⁵: NO₂, CN, CHO, CH₃CO, CH₃OOC,C₂H₅OOC oder CH₃COO und
   - R⁶: H bedeutet;
(f)
   - R⁵: NO₂, CN, CHO, CH₃CO, CH₃OOC,C₂H₅OOC oder CH₃COO bedeutet und in 6-Stellung steht und
   - R⁶: H bedeutet;
(g)
   - R⁵: NO₂ oder CN und
   - R⁶: H bedeutet;
(h)
   - R⁵: NO₂ oder CN bedeutet und in 6-Stellung steht und
   - R⁶: H bedeutet;
(i)
   - R⁵: CN und
   - R⁶: H bedeutet;
(j)
   - R⁵: CN bedeutet und in 6-Stellung steht und
   - R⁶: H bedeutet.

Im übrigen haben vor- und nachstehend die Reste R¹ bis R⁶, Z, A, "-alkyl" und Ac die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I, dadurch gekennzeichnet, daß man eine Verbindung der Formel II 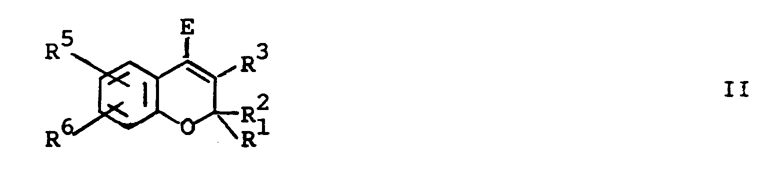
worin
- E: Cl, Br, J oder eine reaktionsfähig veresterte OH-Gruppe bedeutet und
- R¹, R², R³, R⁵, und R⁶: die bei Formel I angegebenen Bedeutungen haben,
mit einer Verbindung der Formel III

R⁴-Z-H III

worin
- R⁴ und Z: die bei Formel I angegebenen Bedeutungen haben,
umsetzt,
oder daß man zur Herstellung einer Verbindung der Formel I, worin R³ = CH₂OH ist, eine Verbindung der Formel I, worin R³ = CHO ist, mit einem reduzierenden Mittel behandelt,
und/oder daß man in einer Verbindung der Formel I einen oder mehrere der Reste R⁴, R⁵ und/oder R⁶ in andere Reste R⁴, R⁵ und/oder R⁶ umwandelt und/oder daß man eine basische bzw. saure Verbindung der Formel I durch Behandeln mit einer Säure bzw. Base in eines ihrer Salze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150°.

Ausgangsstoffe der Formel II sind neu. Diejenigen mit R³ = CHO und E = Br sind bevorzugt. Diese sind herstellbar durch Umsetzung von 2-Hydroxyacetophenonen der Formel 2-HO-R⁵R⁶C₆H₂-COCH₃ mit Ketonen der Formel R¹-CO-R² zu entsprechenden im Benzolring gegebenenfalls durch R⁵ und R⁶ substituierten 2-R¹-2-R²-4-chromanonen und nachfolgende Reaktion derselben mit PBr₃/Dimethylformamid (DMF). Die Verbindungen der Formel II mit R³ = CH₂OH sind daraus durch Reduktion erhältlich.

Die Ausgangsstoffe der Formel III sind in der Regel bekannt (vgl. z.B. DE-OS 37 26 261). Sofern sie nicht bekannt sind, können sie nach an sich bekannten Methoden hergestellt werden.

In Verbindungen der Formel II kommen als "reaktionsfähig veresterte OH-Gruppen" insbesondere die Ester mit Alkylsulfonsäuren (worin die Alkylgruppe 1-6 C-Atome enthält) oder mit Arylsulfonsäuren (worin die Arylgruppe 6-10 C-Atome enthält) in Betracht.

Bei der Umsetzung von II mit III ist es zweckmäßig, in Gegenwart einer Base zu arbeiten. Als Basen eignen sich bevorzugt Alkalimetall- oder Erdalkalimetall-hydroxide, -carbonate, -alkoholate, oder auch -amide wie NaOH, KOH, Ca(OH)₂, Na₂CO₃, K₂CO₃, Na- oder K-methylat, -ethylat oder -tert.-butylat, NaNH₂, KNH₂, ferner organische Basen wie Triethylamin oder Pyridin, die auch im Überschuß angewendet werden und dann gleichzeitig als Lösungsmittel dienen können.

Als inerte Lösungmittel eignen sich insbesondere Alkohole wie Methanol, Ethanol, Isopropanol, n-Butanol oder tert.-Butanol; Ether wie Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Amide wie DMF, Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid. Weiterhin eignen sich Gemische dieser Lösungsmittel untereinander.

Eine besonders bevorzugte Arbeitsweise besteht darin, daß man ein Gemisch von II, III und überschüssigem K₂CO₃ in DMF bei 40-70° umsetzt.

Zur Herstellung von Verbindungen der Formel I, in denen R³ = CH₂OH ist, kann man auch Aldehyde der Formel I, in denen R³ = CHO ist, mit einem reduzierenden Mittel behandeln. Bevorzugt verwendet man ein komplexes Metallhydrid wie NaBH₄ oder LiBH₄ und arbeitet in einem der angegebenen Lösungsmittel, vorzugsweise in einem Alkohol wie Methanol bei Temperaturen zwischen 10 und 50°, vorzugsweise zwischen 15 und 30°.

Weiterhin kann man in einer Verbindung der Formel I einen oder mehrere der Reste R⁴, R⁵ und/oder R⁶ in andere Reste R⁴, R⁵ und/oder R⁶ umwandeln.

Beispielsweise ist es möglich, daß man ein H-Atom mittels einer Halogenierung durch ein Halogenatom ersetzt und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z.B. mit Raney-Nickel in Wasser/Essigsäure/Pyridin in Gegenwart von Natriumphosphat) in eine Formylgruppe oder (z.B. mit KOH in tert.-Butanol) in eine Carbamoylgruppe oder (z.B. mit H₂S in Pyridin/Triethylamin) in eine Thiocarbamoylgruppe umwandelt und/oder eine Carbamoylgruppe (z.B. mit POCl₃) zu einer Cyangruppe dehydratisiert und/oder eine -CO-NH-Gruppe (z.B. mit P₂S₅ oder mit Lawesson-Reagenz in Toluol) in eine -CS-NH- bzw. -C(SH)=N-Gruppe umwandelt.

Eine Halogenierung kann z.B. mit elementarem Chlor oder Brom in einem der üblichen inerten Lösungsmittel bei Temperaturen zwischen 0 und 30° durchgeführt werden. Falls mindestens einer der Substituenten R⁵ und R⁶ eine elektronegative Gruppe wie CN oder NO₂ bedeutet, erfolgt die Chlorierung überwiegend im Rest R⁴; andernfalls erhält man in der Regel Gemische, bei denen die Halogenatome im Rest R⁴ oder am Benzolring stehen können.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z.B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z.B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungemittel, z.B. DMF, bei Temperaturen zwischen 0 ° und 120 ° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z.B. Chloride oder Bromide) oder Anhydride von Carbonsäuren der Formel Ac-OH, z.B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z.B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen 0° und 160°, vorzugsweise zwischen 20° und 120°. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Aufreinigung der Verbindungen der Formel I verwendet werden.

Saure Verbindungen der Formel I können durch Behandeln mit Basen in entsprechende Salze übergeführt werden, z.B. mit NaOH in ihre Na-Salze.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann.

Erhaltene Racemate können, falls erwünscht, nach an sich bekannten Methoden mechanisch, chemisch oder biochemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z.B. optisch aktive Säuren, wie die D- und L-Formen von Weinsäure, Dibenzoylweinsäure, Diacetylweinsäure, Camphansäure, Camphersulfonsäuren, Mandelsäure, Äpfelsäure oder Milchsäure. Carbinole (z.B. I, R³ = CH₂OH) können ferner mit Hilfe chiraler Acylierungsreagenzien, z.B. D- oder L-α-Methylbenzylisocyanat, verestert und dann getrennt werden (vgl. EP-A1-120428). Die verschiedenen Formen der Diastereomeren können in an sich bekannter Weise, z.B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z. B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern, z. B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankrung, welcher die Therapie gilt. Die orale Applikation ist bevorzugt.

Die Verbindungen der Formel I und ihre Salze eignen sich, insbesondere bei topischer Anwendung, ferner zur Behandlung der Alopecia areata. Hierfür werden insbesondere pharmazeutische Zubereitungen verwendet, die zur topischen Behandlung der Kopfhaut geeignet und die oben genannt sind. Sie enthalten 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":
Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

### Beispiel 1

Ein Gemisch von 1,4 g 2,2-Dimethyl-3-formyl-4-brom-6-cyan-3-chromen (F. 124-125°; erhältlich durch Zutropfen einer Lösung von 2,2-Dimethyl-6-cyan-4-chromanon in Chloroform zu einem Gemisch gleicher Volumenteile DMF und PBr₃ in Chloroform und nachfolgendes 11std. Kochen), 0,9 g 1H-2-Pyridon, 3 g K₂CO₃ und 20 ml DMF wird 1 Std. bei 60° gerührt. Man verdünnt mit Ethylacetat, filtriert, arbeitet das Filtrat wie üblich auf und erhält 2,2-Dimethyl-3-formyl-4-(1,2-dihydro-2-oxo-1-pyridyl)-6-cyan-3-chromen ("A"), F. 190-192°.

Analog erhält man:
mit 3,6-Pyrazindiol (= 3-Hydroxy-1,6-dihydro-6-pyridazinon):
2,2-Dimethyl-3-formyl-4-(6-oxo-1,6-dihydro-3-pyridazinyl-oxy)-6-cyan-3-chromen, F. 270-272°
mit 2,4-Dihydroxypyridin:
2,2-Dimethyl-3-formyl-4-(1,2-dihydro-2-oxo-4-pyridyl-oxy)-6-cyan-3-chromen
mit 2-Pyrrolidinon:
2,2-Dimethyl-3-formyl-4-(2-oxo-pyrrolidino)-6-cyan-3-chromen
mit 1,3-Cyclopentandion:
2,2-Dimethyl-3-formyl-4-(3-oxo-1-cyclopentenyl-oxy)-6-cyan-3-chromen.

Analog erhält man aus den entsprechenden 3-Formyl-4-brom-3-chromenen:
2,2-Tetramethylen-3-formyl-4-(1,2-dihydro-2-oxo-1-pyridyl)-6-cyan-3-chromen
2,2-Pentamethylen-3-formyl-4-(1,2-dihydro-2-oxo-1-pyridyl)-6-cyan-3-chromen
2,2-Dimethyl-3-formyl-4-(1,2-dihydro-2-oxo-1-pyridyl)-6-nitro-3-chromen
2,2-Dimethyl-3-formyl-4-(1,2-dihydro-2-oxo-1-pyridyl)-6-brom-3-chromen
2,2-Dimethyl-3-formyl-4-(1,2-dihydro-2-oxo-1-pyridyl)-6-acetyl-3-chromen
2,2-Dimethyl-3-formyl-4-(1,2-dihydro-2-oxo-1-pyridyl)-6-methoxycarbonyl-3-chromen
2,2-Dimethyl-3-formyl-4-(6-oxo-1,6-dihydro-3-pyridazinyl-oxy)-6-nitro-3-chromen
2,2-Dimethyl-3-formyl-4-(6-oxo-1,6-dihydro-3-pyridazinyl-oxy)-6-brom-3-chromen
2,2-Dimethyl-3-formyl-4-(6-oxo-1,6-dihydro-3-pyridazinyl-oxy)-6-acetyl-3-chromen
2,2-Dimethyl-3-formyl-4-(6-oxo-1,6-dihydro-3-pyridazinyl-oxy)-6-methoxycarbonyl-3-chromen
2,2-Dimethyl-3-formyl-4-(1,2-dihydro-2-oxo-4-pyridyl-oxy)-6-nitro-3-chromen
2,2-Dimethyl-3-formyl-4-(1,2-dihydro-2-oxo-4-pyridyl-oxy)-6-brom-3-chromen
2,2-Dimethyl-3-formyl-4-(1,2-dihydro-2-oxo-4-pyridyl-oxy)-6-acetyl-3-chromen
2,2-Dimethyl-3-formyl-4-(1,2-dihydro-2-oxo-4-pyridyl-oxy)-6-methoxycarbonyl-3-chromen
2,2-Dimethyl-3-formyl-4-(2-oxo-pyrrolidino)-6-nitro-3-chromen
2,2-Dimethyl-3-formyl-4-(2-oxo-pyrrolidino)-6-brom-3-chromen
2,2-Dimethyl-3-formyl-4-(2-oxo-pyrrolidino)-6-acetyl-3-chromen
2,2-Dimethyl-3-formyl-4-(2-oxo-pyrrolidino)-6-methoxycarbonyl-3-chromen
2,2-Dimethyl-3-formyl-4-(3-oxo-1-cyclopentenyl-oxy)-6-nitro-3-chromen
2,2-Dimethyl-3-formyl-4-(3-oxo-1-cyclopentenyl-oxy)-6-brom-3-chromen
2,2-Dimethyl-3-formyl-4-(3-oxo-1-cyclopentenyl-oxy)-6-acetyl-3-chromen
2,2-Dimethyl-3-formyl-4-(3-oxo-1-cyclopentenyl-oxy)-6-methoxycarbonyl-3-chromen.

### Beispiel 2

Eine Lösung von 300 mg ("A") in 20 ml Methanol wird mit 200 mg NaBH₄ versetzt. Man dampft ein, arbeitet wie üblich auf und erhält 2,2-Dimethyl-3-hydroxymethyl-4-(1,2-dihydro-2-oxo-1-pyridyl)-6-cyan-3-chromen, F. 190-192°.

Analog erhält man durch Reduktion der entsprechenden 3-Formylverbindungen:
2,2-Dimethyl-3-hydroxymethyl-4-(6-oxo-1,6-dihydro-3-pyridazinyl-oxy)-6-cyan-3-chromen
2,2-Dimethyl-3-hydroxymethyl-4-(1,2-dihydro-2-oxo-4-pyridyl-oxy)-6-cyan-3-chromen
2,2-Dimethyl-3-hydroxymethyl-4-(2-oxopyrrolidino)-6-cyan-3-chromen
2,2-Dimethyl-3-hydroxymethyl-4-(3-oxo-1-cyclopentenyl-oxy)-6-cyan-3-chromen
2,2-Tetramethylen-3-hydroxymethyl-4-(1,2-dihydro-2-oxo-1-pyridyl)-6-cyan-3-chromen
2,2-Pentamethylen-3-hydroxymethyl-4-(1,2-dihydro-2-oxo-1-pyridyl)-6-cyan-3-chromen
2,2-Dimethyl-3-hydroxymethyl-4-(1,2-dihydro-2-oxo-1-pyridyl)-6-nitro-3-chromen
2,2-Dimethyl-3-hydroxymethyl-4-(1,2-dihydro-2-oxo-1-pyridyl)-6-brom-3-chromen
2,2-Dimethyl-3-hydroxymethyl-4-(1,2-dihydro-2-oxo-1-pyridyl)-6-acetyl-3-chromen
2,2-Dimethyl-3-hydroxymethyl-4-(1,2-dihydro-2-oxo-1-pyridyl)-6-methoxycarbonyl-3-chromen
2,2-Dimethyl-3-hydroxymethyl-4-(6-oxo-1,6-dihydro-3-pyridazinyl-oxy)-6-nitro-3-chromen
2,2-Dimethyl-3-hydroxymethyl-4-(6-oxo-1,6-dihydro-3-pyridazinyl-oxy)-6-brom-3-chromen
2,2-Dimethyl-3-hydroxymethyl-4-(6-oxo-1,6-dihydro-3-pyridazinyl-oxy)-6-acetyl-3-chromen
2,2-Dimethyl-3-hydroxymethyl-4-(6-oxo-1,6-dihydro-3-pyridazinyl-oxy)-6-methoxycarbonyl-3-chromen
2,2-Dimethyl-3-hydroxymethyl-4-(1,2-dihydro-2-oxo-4-pyridyl-oxy)-6-nitro-3-chromen
2,2-Dimethyl-3-hydroxymethyl-4-(1,2-dihydro-2-oxo-4-pyridyl-oxy)-6-brom-3-chromen
2,2-Dimethyl-3-hydroxymethyl-4-(1,2-dihydro-2-oxo-4-pyridyl-oxy)-6-acetyl-3-chromen
2,2-Dimethyl-3-hydroxymethyl-4-(1,2-dihydro-2-oxo-4-pyridyl-oxy)-6-methoxycarbonyl-3-chromen
2,2-Dimethyl-3-hydroxymethyl-4-(2-oxo-pyrrolidino)-6-nitro-3-chromen
2,2-Dimethyl-3-hydroxymethyl-4-(2-oxo-pyrrolidino)-6-brom-3-chromen
2,2-Dimethyl-3-hydroxymethyl-4-(2-oxo-pyrrolidino)-6-acetyl-3-chromen
2,2-Dimethyl-3-hydroxymethyl-4-(2-oxo-pyrrolidino)-6-methoxycarbonyl-3-chromen
2,2-Dimethyl-3-hydroxymethyl-4-(3-oxo-1-cyclopentenyl-oxy)-6-nitro-3-chromen
2,2-Dimethyl-3-hydroxymethyl-4-(3-oxo-1-cyclopentenyl-oxy)-6-brom-3-chromen
2,2-Dimethyl-3-hydroxymethyl-4-(3-oxo-1-cyclopentenyl-oxy)-6-acetyl-3-chromen
2,2-Dimethyl-3-hydroxymethyl-4-(3-oxo-1-cyclopentenyl-oxy)-6-methoxycarbonyl-3-chromen.

### Beispiel 3

Eine Lösung von 1 g 2,2-Dimethyl-3-hydroxymethyl-4-(1,2-dihydro-2-oxo-1-pyridyl)-6-nitro-3-chromen in 25 ml Methanol wird bei 20° und 1 bar an 0,5 g 5%igem Pd-C bis zum Stillstand hydriert. Man filtriert, dampft ein und erhält 2,2-Dimethyl-3-hydroxymethyl-4-(1,2-dihydro-2-oxo-1-pyridyl)-6-amino-3-chromen.

### Beispiel 4

Eine Lösung von 1 g 2,2-Dimethyl-3-hydroxymethyl-4-(1,2-dihydro-2-oxo-1-pyridyl)-6-amino-3-chromen in 15 ml Ameisensäure und 1 ml Pyridin wird 24 Std. gekocht und eingedampft. Nach üblicher Aufarbeitung erhält man 2,2-Dimethyl-3-hydroxymethyl-4-(1,2-dihydro-2-oxo-1-pyridyl)-6-formamido-3-chromen.

### Beispiel 5

Ein Gemisch von 1 g 2,2-Dimethyl-3-hydroxymethyl-4-2-dihydro-2-oxo-1-pyridyl)-6-amino-3-chromen, 10 ml Acetanhydrid und 10 ml Pyridin wird 24 Std. bei 20° stehengelassen. Man dampft ein, arbeitet wie üblich auf und erhält 2,2-Dimethyl-3-hydroxymethyl-4-(1,2-dihydro-2-oxo-1-pyridyl)-6-acetamido-3-chromen.

### Beispiel 6

In eine siedende Lösung von 1 g "A" in 50 ml Methanol und 2 ml Wasser wird 12 Std. unter Rühren HCl eingeleitet. Man läßt erkalten und über Nacht stehen. Die ausgefallene 2,2-Dimethyl-3-formyl-4-(1,2-dihydro-2-oxo-1-pyridyl)-3-chromen-6-carbonsäure wird abfiltriert.

### Beispiel 7

Ein Gemisch von 1 g "A", 10 g Trinatriumphosphat-dodekahydrat, 9 ml Pyridin, 9 ml Wasser, 22 ml Essigsäure und 8 g Raney-Nickel (wasserfeucht) wird bei 20° 3 Std. gerührt. Man filtriert, arbeitet wie üblich auf und erhält 2,2-Dimethyl-3,6-diformyl-4-(1,2-dihydro-2-oxo-1-pyridyl)-3-chromen.

### Beispiel 8

Man löst 1 g "A" in 15 ml tert.-Butanol und gibt unter Rühren und Einleiten von N₂ 2 g gepulvertes KOH hinzu. Nach 1std. Kochen und üblicher Aufarbeitung erhält man 2,2-Dimethyl-3-formyl-4-(1,2-dihydro-2-oxo-1-pyridyl)-6-carbamoyl-3-chromen.

### Beispiel 9

In eine Lösung von 1 g "A" in einem Gemisch von 7 ml Pyridin und 7 ml Triethylamin leitet man 3 Std. bei 20° H₂S ein, dampft ein, arbeit wie üblich auf und erhält 2,2-Dimethyl-3-formyl-4-(1,2-dihydro-2-oxo-1-pyridyl)-6-thiocarbamoyl-3-chromen.

Die nachstehenden Beispiele betreffen pharmazeutische Zubereitungen, welche Verbindungen der Formel I und/oder deren physiologisch unbedenkliche Salze enthalten.

### Beispiel 1: Tabletten

Ein Gemisch von 0,2 kg "A", 136,3 kg Calciumhydrogenphosphat, 15 kg Maisstärke, 10 kg mikrokristalliner Cellulose, 5,5 kg unlöslichem Polyvinylpyrrolidon (PVP), 1,5 kg hochdispersem Siliciumdioxid und 1,5 kg Magnesiumstearat wird in üblicher Weise zu Tabletten gepreßt. Jede 170-mg-Tablette enthält 0,2 mg Wirkstoff.

### Beispiel 2: Dragees

Analog Beispiel 1, jedoch ohne den Zusatz von PVP, werden Tabletten gepreßt, die anschließend in üblicher Weise mit einem Überzug aus Saccharose, Maisstärke, Talk, Tragant und Farbstoff überzogen werden.

### Beispiel 3: Lacktabletten

Aus 0,2 kg 2,2-Dimethyl-(3-hydroxymethyl-4-(1,2-dihydro-2-oxo-1-pyridyl)-6-cyan-3-chromen, 151,3 kg Lactose, 10 kg mikrokristalliner Cellulose, 5,5 kg unlöslichem PVP, 1,5 kg hochdispersem Siliciumdioxid und 1,5 kg Calciumstearat werden Tablettenkerne (170 mg) gepreßt, die anschließend in üblicher Weise lackiert werden, so daß jede Lacktablette mit 3,922 mg eines Lackes überzogen ist, der aus 2,2 mg Hydroxypropylmethylcellulose, 0,53 mg Polyethylenglykol 400, 0,85 mg Titandioxid, 0,12 mg Eisen(III)-oxid (gelb), 0,002 mg Eisen(III)-oxid (rot) und 0,22 mg Silikonöl besteht.

### Beispiel 4: Kapseln

Man bereitet ein Granulat aus 10 g 2,2-Dimethyl-3-formyl-4-(6-oxo-1,6-dihydro-3-pyridazinyl-oxy)-6-cyan-3-chromen, 27,5 kg Lactose, 0,35 kg Hydroxypropylmethylcellulose und 0,7 kg Maisstärke, mischt dieses mit 0,15 kg hochdispersem Siliciumdioxid und 0,3 kg Magnesiumstearat und füllt das Gemisch in üblicher Weise in Hartgelatinekapseln, so daß jede Kapsel 0,1 mg Wirkstoff enthält.

### Beispiel 5: Ampullen

Eine Lösung von 10 g "A" in 70 l 1,2-Propandiol wird mit zweifach destilliertem Wasser auf 100 l aufgefüllt, steril filtriert, und in 1 ml-Ampullen abgefüllt, die dann steril verschlossen werden. Jede Ampulle enthält 0,1 mg Wirkstoff.

## Patentansprüche (Patentansprüche für folgende(n) Vertragsstaat(en): AT, BE, CH, LI, DE, DK, FR, GB, GR, IT, LU, NL, SE)

1. Chromanderivate der Formel I 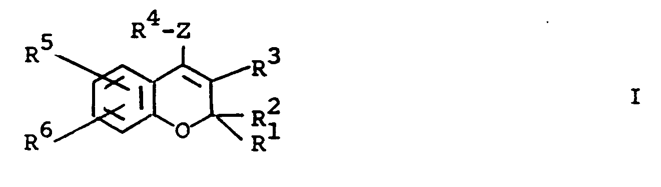 worin
R¹ A,
R² H oder A,
R¹ und R² zusammen auch Alkylen mit 3-6 C-Atomen,
R³ CHO oder CH₂OH,
R⁴ einen unsubstituierten oder ein- oder zweifach durch A, F, Cl, Br, J, OH, OA, OAc, SH, NO₂, NH₂, AcNH, COOH und/oder COOA substituierten Pyridyl-, Pyridazinyl-, Pyrimidinyl-, Pyrazinyl-, Oxo-dihydro-pyridyl-, Oxo-dihydro-pyridazinyl-, Oxo-dihydro-pyrimidinyl-, Oxo-dihydro-pyrazinyl-, 2-Pyrrolidinon-1-yl- oder 3-Oxo-cyclopenten-1-yl-rest,
R⁵ und R⁶ jeweils H, A, HO, AO, CHO, ACO, ACS, HOOC, AOOC, AO-CS, ACOO, A-CS-O, Hydroxy-alkyl, Mercapto-alkyl, NO₂, NH₂, NHA, NA₂, CN, F, Cl, Br, J, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitro-alkyl, Cyan-alkyl, A-C(=NOH) oder A-C(=NNH₂),
Z O, S, NH oder eine Bindung,
A Alkyl mit 1-6 C-Atomen,
-alkyl Alkylen mit 1-6 C-Atomen und
Ac Alkanoyl mit 1-8 C-Atomen oder Aroyl mit 7-11 C-Atomen
bedeuten,
sowie deren Salze.

2. a) 2,2-Dimethyl-3-formyl-4-(1,2-dihydro-2-oxo-1-pyridyl)-6-cyan-3-chromen;
b) 2,2-Dimethyl-3-hydroxymethyl-4-(1,2-dihydro-2-oxo-1-pyridyl)-6-cyan-3-chromen;
c) 2,2-Dimethyl-3-formyl-4-(6-oxo-1,6-dihydro-3-pyridazinyl-oxy)-6-cyan-3-chromen.

3. Verfahren zur Herstellung von Chromanderivaten der Formel I gemäß Anspruch 1, dadurch gekennzeichnet, daß man eine Verbindung der Formel II 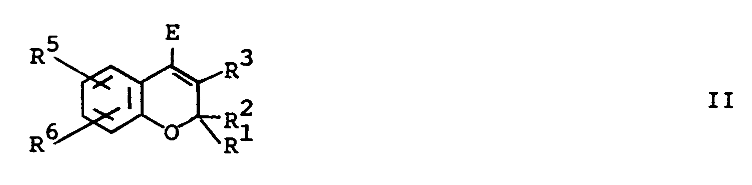 worin
E Cl, Br, J oder eine reaktionsfähig veresterte OH-Gruppe bedeutet und
R¹, R², R³, R⁵, und R⁶ die bei Formel I angegebenen Bedeutungen haben,
mit einer Verbindung der Formel III
R⁴-Z-H III
worin
R⁴ und Z die bei Formel I angegebenen Bedeutungen haben,
umsetzt,
oder daß man zur Herstellung einer Verbindung der Formel I, worin R³ = CH₂OH ist, eine Verbindung der Formel I, worin R³ = CHO ist, mit einem reduzierenden Mittel behandelt,
und/oder daß man in einer Verbindung der Formel I einen oder mehrere der Reste R⁴, R⁵ und/oder R⁶ in andere Reste R⁴, R⁵ und/oder R⁶ umwandelt und/oder daß man eine basische bzw. saure Verbindung der Formel I durch Behandeln mit einer Säure bzw. Base in eines ihrer Salze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I und/oder ihren physiologisch unbedenklichen Salzen zur Herstellung eines Arzneimittels.

## Patentansprüche (Patentansprüche für folgende(n) Vertragsstaat(en): ES)

1. Verfahren zur Herstellung von Chromanderivaten der Formel I 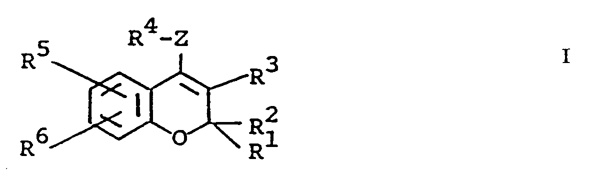 worin
R¹ A,
R² H oder A,
R¹ und R² zusammen auch Alkylen mit 3-6 C-Atomen,
R³ CHO oder CH₂OH,
R⁴ einen unsubstituierten oder ein- oder zweifach durch A, F, Cl, Br, I, OH, OA, OAc, SH, NO₂, NH₂, AcNH, COOH und/oder COOA substituierten Pyridyl-, Pyridazinyl-, Pyrimidinyl-, Pyrazinyl-, Oxo-dihydropyridyl-, Oxo-dihydropyridazinyl-, Oxo-dihydropyrimidinyl- oder Oxo-dihydropyrazinyl-, 2-Pyrrolidinon-1-yl- oder 3-Oxo-cyclopenten-1-yl-rest,
R⁵ und R⁶ jeweils H, A, HO, AO, CHO, ACO, ACS, HOOC, AOOC, AO-CS, ACOO, A-CS-O, Hydroxy-alkyl, Mercapto-alkyl, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitro-alkyl, Cyan-alkyl, A-C(=NOH) oder A-C(=NNH₂),
Z O, S, NH oder eine Bindung,
A Alkyl mit 1-6 C Atomen,
-alkyl Alkylen mit 1-6 C Atomen und
Ac Alkanoyl mit 1-8 C-Atomen oder Aroyl mit 7-11 C-Atomen
bedeuten, sowie deren Salze, dadurch gekennzeichnet,
daß man eine Verbindung der Formel II 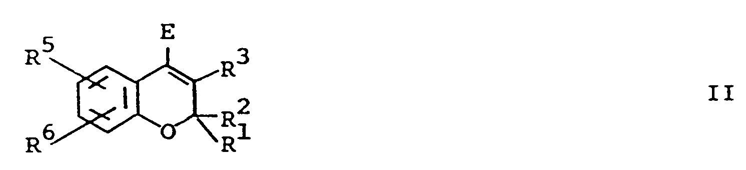 worin
E Cl, Br, J oder eine reaktionsfähig veresterte OH-Gruppe bedeutet und
R¹, R², R³, R⁵, und R⁶ die bei Formel I angegebenen Bedeutungen haben,
mit einer Verbindung der Formel III
R⁴-Z-H III
worin
R⁴ und Z die bei Formel I angegebenen Bedeutungen haben,
umsetzt,
oder daß man zur Herstellung einer Verbindung der Formel I, worin R³ = CH₂OH ist, eine Verbindung der Formel I, worin R³ = CHO ist, mit einem reduzierenden: Mittel behandelt,
und/oder daß man in einer Verbindung der Formel I einen oder mehrere der Reste R⁴, R⁵ und/oder R⁶ in andere Reste R⁴, R⁵ und/oder R⁶ umwandelt und/oder daß man eine basische bzw. saure Verbindung der Formel I durch Behandeln mit einer Säure bzw. Base in eines ihrer Salze umwandelt.

2. Verfahren zur Herstellung von
a) 2,2-Dimethyl-3-formyl-4-(1,2-dihydro-2-oxo-1-pyridyl)-6-cyan-3-chromen;
b) 2,2-Dimethyl-3-hydroxymethyl-4-(1,2-dihydro-2-oxo-1-pyridyl)-6-cyan-3-chromen;
c) 2,2-Dimethyl-3-formyl-4-(6-oxo-1,6-dihydro-3-pyridazinyl-oxy)-6-cyan-3-chromen.
gemäß Anspruch 1.

3. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

## Claims (Claims for the following Contracting State(s): AT, BE, CH, LI, DE, DK, FR, GB, GR, IT, LU, NL, SE)

1. Chroman derivatives of the formula I 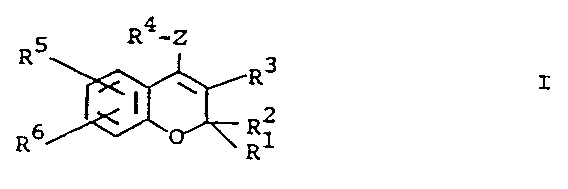 in which
R¹ is A,
R² is H or A,
R¹ and R² together are also alkylene having 3-6 C atoms,
R³ is CHO or CH₂OH,
R⁴ is a pyridyl, pyridazinyl, pyrimidinyl, pyrazinyl, oxodihydropyridyl, oxodihydropyridazinyl, oxodihydropyrimidinyl, oxodihydropyrazinyl, 2-pyrrolidinon-1-yl or 3-oxocyclopenten-1-yl radical which is unsubstituted, monosubstituted or disubstituted by A, F, Cl, Br, I, OH, OA, OAc, SH, NO₂, NH₂, AcNH, COOH and/or COOA,
R⁵ and R⁶ are each H, A, HO, AO, CHO, ACO, ACS, HOOC, AOOC, AO-CS, ACOO, A-CS-O, hydroxyalkyl, mercaptoalkyl, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, nitroalkyl, cyanoalkyl, A-C(=NOH) or A-C(=NNH₂),
Z is O, S, NH or a bond,
A is alkyl having 1-6 C atoms,
-alkyl is alkylene having 1-6 C atoms and
Ac is alkanoyl having 1-8 C atoms or aroyl having 7-11 C atoms
and their salts.

2. a) 2,2-Dimethyl-3-formyl-4-(1,2-dihydro-2-oxo-1-pyridyl)-6-cyano-3-chromene,
b) 2,2-Dimethyl-3-hydroxymethyl-4-(1,2-dihydro-2-oxo-1-pyridyl)-6-cyano-3-chromene;
c) 2,2-Dimethyl-3-formyl-4-(6-oxo-1,6-dihydro-3-pyridazinyloxy)-6-cyano-3-chromene.

3. Process for the preparation of chroman derivatives of the formula I according to Claim 1, characterized in that a compound of the formula II 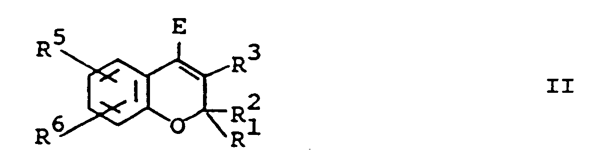 in which
E is Cl, Br, I or a reactively esterified OH group and
R¹, R², R³, R⁵ and R⁶ have the meanings given in formula I
is reacted with a compound of the formula III
R⁴-Z-H III
in which
R⁴ and Z have the meanings indicated in formula I,
or in that for the preparation of a compound of the formula I in which R³ is CH₂OH, a compound of the formula I in which R³ is CHO is treated with a reducing agent, and/or in that in a compound of the formula I, one or more of the radicals R⁴, R⁵, and/or R⁶ are converted into other radicals R⁴, R⁵, and/or R⁶ and/or in that a basic or acidic compound of the formula I is converted into one of its salts by treating with an acid or base.

4. Process for the production of pharmaceutical preparations, characterized in that a compound of the formula I and/or one of its physiologically acceptable salts is brought into a suitable form for administration together with at least one solid, liquid or semi-liquid excipient or auxiliary.

5. Pharmaceutical preparation which contains at least one compound of the formula I and/or one of its physiologically acceptable salts.

6. Use of compounds of the formula I and/or their physiologically acceptable salts for the production of a medicament.

## Claims (Claims for the following Contracting State(s): ES)

1. Process for the preparation of chroman derivatives of the formula I 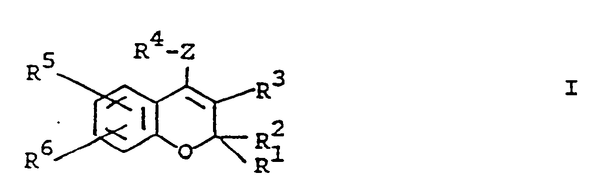 in which
R¹ is A,
R² is H or A,
R¹ and R² together are also alkylene having 3-6 C atoms,
R³ is CHO or CH₂OH,
R⁴ is a pyridyl, pyridazinyl, pyrimidinyl, pyrazinyl, oxodihydropyridyl, oxodihydropyridazinyl, oxodihydropyrimidinyl or oxodihydropyrazinyl, 2-pyrrolidinon-1-yl or 3-oxocyclopenten-1-yl radical which is unsubstituted, monosubstituted or disubstituted by A, F, Cl, Br, I, OH, OA, OAc, SH, NO₂, NH₂, AcNH, COOH and/or COOA,
R⁵ and R⁶ are each H, A, HO, AO, CHO, ACO, ACS, HOOC, AOOC, AO-CS, ACOO, A-CS-O, hydroxyalkyl, mercaptoalkyl, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, nitroalkyl, cyanoalkyl, A-C(=NOH) or A-C(=NNH₂),
Z is O, S, NH or a bond,
A is alkyl having 1-6 C atoms,
-alkyl is alkylene having 1-6 C atoms and
Ac is alkanoyl having 1-8 C atoms or aroyl having 7-11 C atoms
and their salts, characterized in that a compound of the formula II 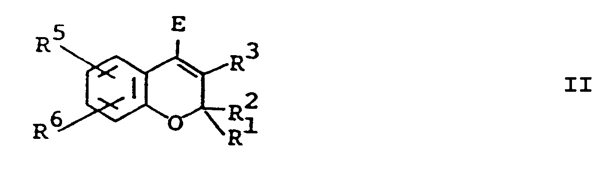 in which
E is Cl, Br, I or a reactively esterified OH group and
R¹, R², R³, R⁵ and R⁶ have the meanings given in formula I
is reacted with a compound of the formula III
R⁴-Z-H III
in which
R⁴ and Z have the meanings indicated in formula I,
or in that for the preparation of a compound of the formula I in which R³ is CH₂OH, a compound of the formula I in which R³ is CHO is treated with a reducing agent, and/or in that in a compound of the formula I, one or more of the radicals R⁴, R⁵, and/or R⁶ are converted into other radicals R⁴, R⁵, and/or R⁶ and/or in that a basic or acidic compound of the formula I is converted into one of its salts by treating with an acid or base.

2. Process for the preparation of
a) 2,2-Dimethyl-3-formyl-4-(1,2-dihydro-2-oxo-1-pyridyl)-6-cyano-3-chromene,
b) 2,2-Dimethyl-3-hydroxymethyl-4-(1,2-dihydro-2-oxo-1-pyridyl)-6-cyano-3-chromene;
c) 2,2-Dimethyl-3-formyl-4-(6-oxo-1,6-dihydro-3-pyridazinyloxy)-6-cyano-3-chromene
according to claim 1.

3. Process for the production of pharmaceutical preparations, characterized in that a compound of the formula I and/or one of its physiologically acceptable salts is brought into a suitable form for administration together with at least one solid, liquid or semi-liquid excipient or auxiliary.

## Revendications (Revendications pour l'(les) Etat(s) contractant(s) suivant(s): AT, BE, CH, LI, DE, DK, FR, GB, GR, IT, LU, NL, SE)

1. Dérivés du chromane répondant à la formule I 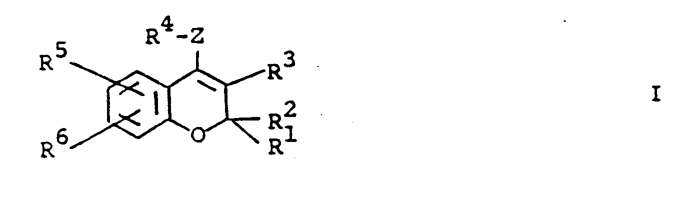 dans laquelle
R¹ représente A,
R² représente H ou A,
R¹ et R² peuvent également former ensemble un groupe alkylène en C 3-C 6,
R³ représente CHO ou CH₂OH,
R⁴ représente un groupe pyridyle, pyridazinyle, pyrimidinyle, pyrazinyle, oxo-dihydro-pyridyle, oxo-dihydro-pyridazinyle, oxo-dihydro-pyrimidinyle, oxo-dihydro-pyrazinyle, 2-pyrrolidinone-1-yle ou 3-oxo-cyclopentène-1-yle non substitué ou portant un ou deux substituants choisis parmi A, F, Cl, Br, I, OH, OA, OAc, SH, NO₂, NH₂, AcNH, COOH et/ou COOA,
R⁵ et R⁶ représentent chacun H, A, un groupe HO, AO, CHO, ACO, ACS, HOOC, AOOC, AO-CS, ACOO, A-CS-O, hydroxy-alkyle, mercapto-alkyle, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyle, nitro-alkyle, cyan-alkyle, A-C(=NOH) ou A-C(=NNH₂),
Z représente O, S, NH ou une liaison,
A représente un groupe alkyle en C 1-C 6,
-alkyle représente un groupe alkylène en C 1-C 6, et
Ac représente un groupe alcanoyle en C 1-C 8 ou aroyle en C 7-C 11,
et leurs sels.

2. a) Le 2,2-diméthyl-3-formyl-4-(1,2-dihydro-2-oxo-1-pyridyl)-6-cyano-3-chromène;
b) le 2,2-diméthyl-3-hydroxyméthyl-4-(1,2-dihydro-2-oxo-1-pyridyl)-6-cyano-3-chromène;
c) le 2,2-diméthyl-3-formyl-4-(6-oxo-1,6-dihydro-3-pyridazinyl-oxy)-6-cyano-3-chromène.

3. Procédé de préparation des dérivés du chromane de formule I de la revendication 1, caractérisé en ce que l'on fait réagir un composé de formule II 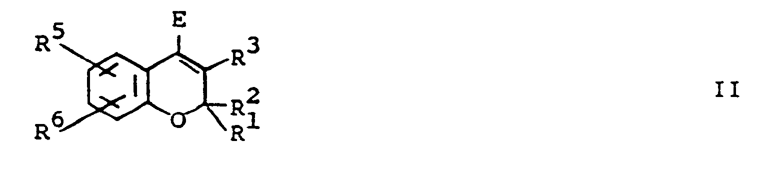 dans laquelle
E représente Cl, Br, I ou un groupe OH estérifié réactif, et
R¹, R², R³, R⁵ et R⁶ ont les significations indiquées en référence à la formule I,
avec un composé de formule III
R⁴-Z-H III
dans laquelle R⁴ et Z ont les significations indiquées en référence à la formule I,
ou bien, pour préparer un composé de formule I dans laquelle R³ = CH₂OH, on traite par un agent réducteur un composé de formule I dans laquelle R³ = CHO,
et/ou, dans un composé de formule I, on convertit un ou plusieurs des substituants R⁴, R⁵ et/ou R⁶ en d'autres substituants R⁴, R⁵ et/ou R⁶, et/ou on convertit un composé de formule I basique ou respectivement acide par traitement à l'aide d'un acide ou d'une base respectivement, en l'un de ses sels.

4. Procédé de préparation de compositions pharmaceutiques, caractérisé en ce que l'on met sous une forme de dosage appropriée un composé de formule I et/ou l'un de ses sels acceptables pour l'usage pharmaceutique avec au moins un véhicule ou produit auxiliaire solide, liquide ou semi-liquide.

5. Composition pharmaceutique, caractérisée en ce qu'elle contient au moins un composé de formule I et/ou un de ses sels acceptables pour l'usage pharmaceutique.

6. Utilisation des composés de formule I et/ou de leurs sels acceptables pour l'usage pharmaceutique pour la préparation d'un médicament.

## Revendications (Revendications pour l'(les) Etat(s) contractant(s) suivant(s): ES)

1. Procédé de préparation des dérivés du chromane répondant à la formule I 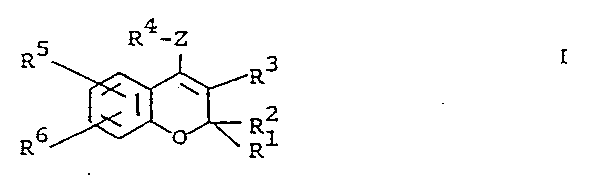 dans laquelle
R¹ représente A,
R² représente H ou A,
R¹ et R² peuvent également former ensemble un groupe alkylène en C 3-C 6,
R³ représente CHO ou CH₂OH,
R⁴ représente un groupe pyridyle, pyridazinyle, pyrimidinyle, pyrazinyle, oxo-dihydro-pyridyle, oxo-dihydro-pyridazinyle, oxo-dihydro-pyrimidinyle, oxo-dihydro-pyrazinyle, 2-pyrrolidinone-1-yle ou 3-oxo-cyclopentène-1-yle non substitué ou portant un ou deux substituants choisis parmi A, F, Cl, Br, I, OH, OA, OAc, SH, NO₂, NH₂, AcNH, COOH et/ou COOA,
R⁵ et R⁶ représentent chacun H, A, un groupe HO, AO, CHO, ACO, ACS, HOOC, AOOC, AO-CS, ACOO, A-CS-O, hydroxy-alkyle, mercapto-alkyle, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyle, nitro-alkyle, cyan-alkyle, A-C(=NOH) ou A-C(=NNH₂),
Z représente O, S, NH ou une liaison,
A représente un groupe alkyle en C 1-C 6,
- alkyle représente un groupe alkylène en C 1-C 6, et
Ac représente un groupe alcanoyle en C 1-C 8 ou aroyle en C 7-C 11,
et de leurs sels, caractérisé en ce que l'on fait réagir un composé de formule II 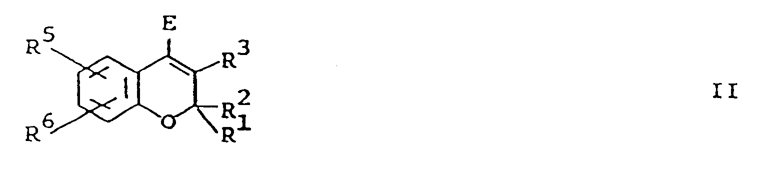 dans laquelle
E représente Cl, Br, I ou un groupe OH estérifié réactif, et
R¹, R², R³, R⁵ et R⁶ ont les significations indiquées en référence à la formule I,
avec un composé de formule III
R⁴-Z-H III
dans laquelle R⁴ et Z ont les significations indiquées en référence à la formule I,
ou bien, pour préparer un composé de formule I dans laquelle R³ = CH₂OH, on traite par un agent réducteur un composé de formule I dans laquelle R³ = CHO,
et/ou, dans un composé de formule I, on convertit un ou plusieurs des substituants R⁴, R⁵ et/ou R⁶ en d'autres substituants R⁴, R⁵ et/ou R⁶, et/ou on convertit un composé de formule I basique ou respectivement acide par traitement à l'aide d'un acide ou d'une base respectivement, en l'un de ses sels.

2. Procédé de préparation de :
a) 2,2-diméthyl-3-formyl-4-(1,2-dihydro-2-oxo-1-pyridyl)-6-cyano-3-chromène;
b) 2,2-diméthyl-3-hydroxyméthyl-4-(1,2-dihydro-2-oxo-1-pyridyl)-6-cyano-3-chromène;
c) 2,2-diméthyl-3-formyl-4-(6-oxo-1,6-dihydro-3-pyridazinyl-oxy)-6-cyano-3-chromène,
selon revendication 1.

3. Procédé de préparation de compositions pharmaceutiques, caractérisé en ce que l'on met sous une forme de dosage appropriée un composé de formule I et/ou l'un de ses sels acceptables pour l'usage pharmaceutique avec au moins un véhicule ou produit auxiliaire solide, liquide ou semi-liquide.
